# EUROPEAN PATENT APPLICATION

(11) **EP 4 643 821 A1**
(43) Date of publication of application: **05.11.2025**
(21) Application number: 24861317.6
(22) Date of filing: 14.03.2024
(51) Int. Cl.: A61F 2/28, A61F 2/30

(54) **RIB PROSTHESIS HAVING CONTROLLED ELASTICITY**

(71) Applicant: Nsilica Simulation Technologies, S.L., 15229 Ames (a Coruña) (ES)
(72) Inventor: LÓPEZ CAMPOS, José Ángel, 36211 Vigo (Pontevedra) (ES); SEGADE ROBLEDA, Abraham, 36203 Vigo (Pontevedra) (ES); SUÁREZ GARCÍA, Sofía, 15229 Ames (A Coruña) (ES)
(74) Representative: González López-Menchero, Álvaro Luis
(86) International application number: PCT/ES2024/070160
(87) International publication number: WO 2025/191187

(57) **Abstract**

The present invention relates to a rib prosthesis with controlled elasticity that comprises an front end (1) and a rear end (2) arranged opposite each other and, between them, an intermediate elastic structure capable of absorbing pressure in all directions and consisting of an array (8) of unit cells (3) with an inner cavity (4), the unit cells being joined to the adjacent cells by means of intermediate attachment points (7), by means of upper attachment points (5) and other lower attachment points (6). Each of the unit cells (3) are formed by a first arm (3.3) and a second arm (3.4) opposite each other in such a way that a central space (3.5) is defined and, depending on the thickness of the arms and on the angle formed by the arms, they will have more or less elasticity. It is possible to form a prosthesis for a set of ribs when they are joined by a common fixing element.

## Description

### OBJECT OF THE INVENTION

The object of the present invention, as established by the title of the invention, is a rib prosthesis with controlled elasticity, in other words, it refers to a part used to replace one or more sections of biological rib or a set of ribs or even to form a thoracic prosthesis that allows a part of the rib cage to be reconstructed. The prosthesis has the particularity of having a controlled elasticity so that the structure of the prosthesis can be adjusted to have the same stiffness as the set of biological tissue replaced.

The present invention is characterised by the special design and configuration of each of the individual rib-forming cells that make it possible for the prosthesis to exhibit controlled elasticity.

Therefore, the present invention falls within the field of body prostheses, and in particular rib prostheses for rib cage reconstruction.

### BACKGROUND OF THE INVENTION

Currently, the most common rib prostheses are structures with a simple design, which only emulate the geometry of the ribs and try to maintain the geometry of the rib cage. In some cases, however, they do not even reach that level of detail and can become highly invasive elements that in no way emulate either the geometry or the real behaviour of human ribs.

Current prostheses have a strong orientation towards the replacement of a relatively large set of ribs. However, in some interventions, the ability to replace a small number of ribs, a single rib, or even a limited rib fragment may be of interest.

The replacement of one or more ribs with a mechanical element should not be seen as a purely aesthetic process or one in which only the geometry plays a fundamental role; the ribs have an elastic behaviour that facilitates the patient's ventilatory mechanics.

When replacing a rib with a mechanical component of greater or lesser stiffness, not only are the ventilatory mechanics affected and may lead to breathing difficulties or discomfort, but phenomena such as stress shielding could also appear, which is the reduction in bone density due to the implant taking on most of the stress and inhibiting the natural mechanotransduction mechanisms.

The ribs, in addition to their ventilatory function, have a protective function for protecting the internal organs. That is why the ribs generally have areas with very different local stiffness, where the stiffness will typically be lower closer to the sternum, and as we get closer to the spine, the stiffness will be much greater, with the protective function prevailing.

Furthermore, patent CN109199644A is known in the prior art and discloses a biomimetic costal cartilage prosthesis implant and a method of preparing the same, wherein said costal cartilage prosthesis implant has the integral form of a strip, comprises an intermediate elastic structure and a two-ended fixation structure, wherein the elastic structure in the middle of the costal cartilage prosthesis is a mechanical corrugated structure, a spiral structure, and the elastic coefficient of the costal cartilage prosthesis can be changed according to different positions of the implant by changing the parameters of the intermediate elastic structure. The costal cartilage prosthesis implant has two end fixation structures, corresponding ribs and sternum, and has a structure for mounting fixation substitutes. The rib and costal cartilage implant, developed as a result of the innovation, aims to replicate the mechanical properties of the original tissue. Its main objective is to improve the physiological function of the sternal rib prosthesis after placement during bone reconstruction surgery of the chest wall. This implant seeks to effectively reduce interference with the respiratory function after surgery and have a positive impact on patients' postoperative recovery.

Therefore, the object of the present invention is to overcome the difficulties mentioned above, in other words, an invention that can be used as a unit or in combination with other similar elements, that has multidirectional elasticity, as well as a certain resistance so that it offers protection to the internal organs, developing a rib prosthesis to replace one or more sections of biological rib or a set of ribs or even to form a thoracic prosthesis as described below and as essentially disclosed in the first claim.

### DESCRIPTION OF THE INVENTION

The object of the present invention is essentially disclosed in the independent claim and the various embodiments are disclosed in the dependent claims.

The present invention relates to a rib prosthesis with controlled elasticity formed from a series of unit cells repeated along the geometry of the prosthesis, wherein said unit cells are joined together to form an array, by way of a tissue, forming a geometry with an internal cavity, with solid prosthesis ends, and able to be fixed to the native bone by means of conventional methods.

The formed array and the subsequent geometry adopted allows stresses to be absorbed through the deformation of the alveolar network formed by the unit cells.

The features described above make it possible to achieve the following:
- Generate prostheses for any number of ribs, also including the possibility of generating unitary and reduced rib fragment prostheses.
- It allows the different stiffnesses in the different areas to be addressed, adapting and imitating the geometry of the rib cage as well as imitating its elasticity and adapting to the patient's ventilatory mechanics.

The manufacturing material may be any of the known materials, the materials of the end fixation brackets and of the structure not necessarily being the same.

Unless otherwise indicated, all technical and scientific elements used herein have the meaning that is usually understood by a person with skill in the art to which this invention belongs. Methods and materials similar or equivalent to those described in the specification may be used in the practice of the present invention.

Throughout the description and in the claims, the word "comprises" and its variants are not intended to exclude other technical features, additives, components or steps. For those skilled in the art, other objects, advantages and features of the invention may be deduced from both the description and the practical use of the invention.

### EXPLANATION OF THE FIGURES

As a complement to the description provided below, and for the purpose of helping to make the features of the invention more readily understandable, in accordance with a practical preferred exemplary embodiment thereof, said description is accompanied by a set of drawings which, by way of illustration and not limitation, represent the following.
Figure 1 shows a front view representation of the rib prosthesis object of the invention.
Figure 2 shows a perspective representation of the geometry of the rib prosthesis object of the application.
Figure 3 shows a detail of a unit cell forming the rib prosthesis.
Figures 4 and 5 show the concept of stiffness variation using two sets of six unit cells with different thicknesses and which will therefore have different overall stiffness.

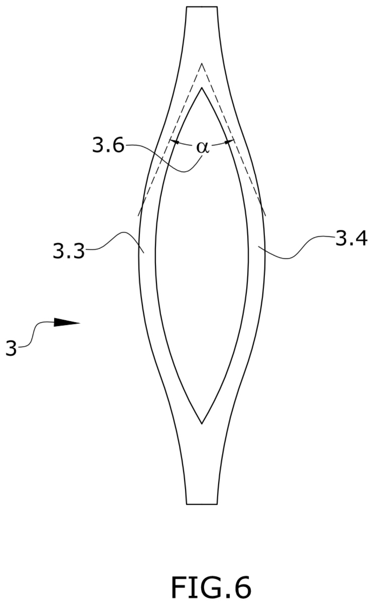

Figure 6 shows a unit cell and highlights the angle which variation results in a variation of the elasticity of the assembly.
Figures 7 and 8 show two arrays formed by different cells, which together have different stiffness.
Figure 9 shows a gradually stiffened rib generated using thinner cells close to the end 2 and thicker cells close to the end 1.
Figure 10 shows the concept of a prosthesis for more than one rib with attachment to the sternum.

### PREFERRED EMBODIMENT OF THE INVENTION

In light of the figures, a preferred embodiment of the proposed invention is described below.

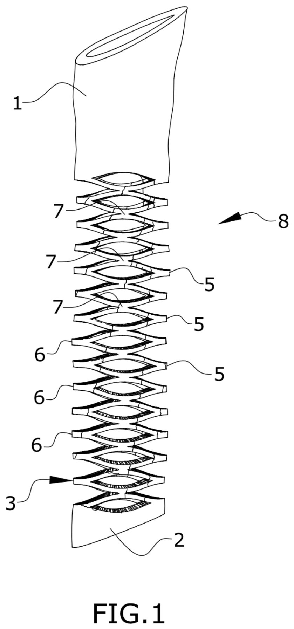

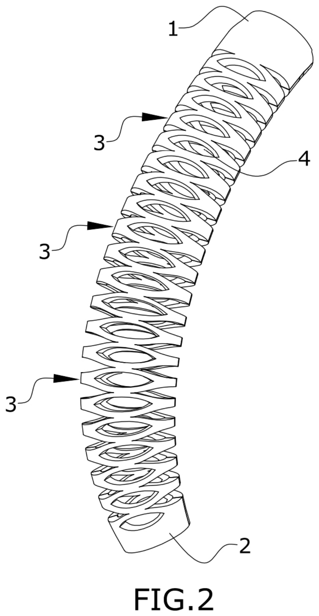

Figure 1 shows that the rib prosthesis object of the invention comprises a front end (1) and a rear end (2) arranged opposite each other and, between them, an intermediate elastic structure capable of absorbing stresses in all directions and consisting of an array (8) of unit cells (3). The configuration of said array is such that there is a hollow inner cavity (4) (figure 2), the unit cells being joined to the adjacent cells by means of intermediate attachment points (7), by means of upper attachment points (5) and other lower attachment points (6).

The front end (1) and the rear end (2) act as fixation structures to the rest of the skeleton.

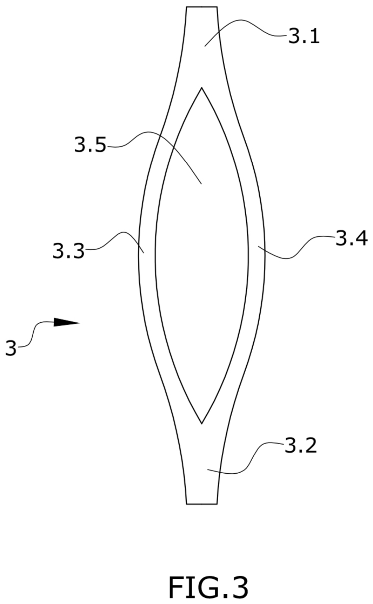

As shown in figure 3, each of the unit cells (3), seen from a flat configuration, is formed by a first arm (3.3) and a second arm (3.4) opposite each other in such a way that a central space (3.5) is defined, the upper ends of the first arm (3.3) and of the second arm (3.4) being joined to form an upper joint (3.1), while the lower ends of the first arm (3.3) and of the second arm (3.4) are joined to form a lower joint (3.2).

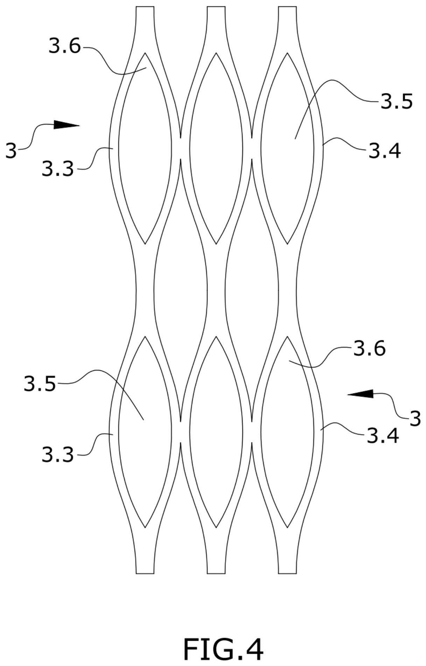

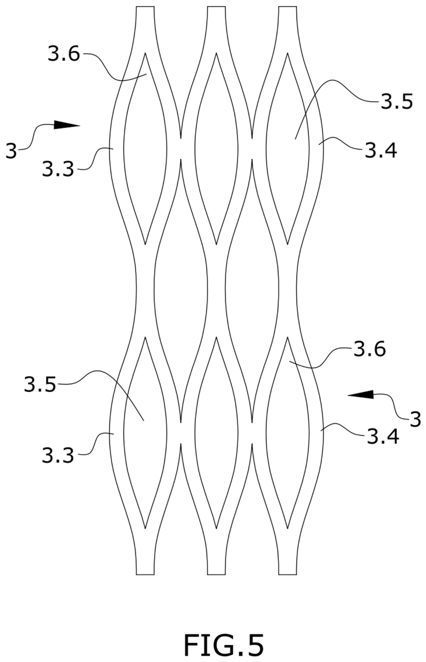

Figures 4 and 5 show structures formed by unit cells (3) with different stiffness, given that the unit cells (3) shown in figure 4 have a thickness of the arms (3.3) and (3.4) less than the thickness of the arms (3.3) and (3.5) of the unit cells shown in figure 5. Furthermore, the internal angle (3.6) defined at the ends of the central spaces (3.5) is smaller in the unit cells (3) in figure 4 than in the unit cells (3) in figure 5, the unit cells (3) in figure 4 having less stiffness than the unit cells (3) in figure 5.

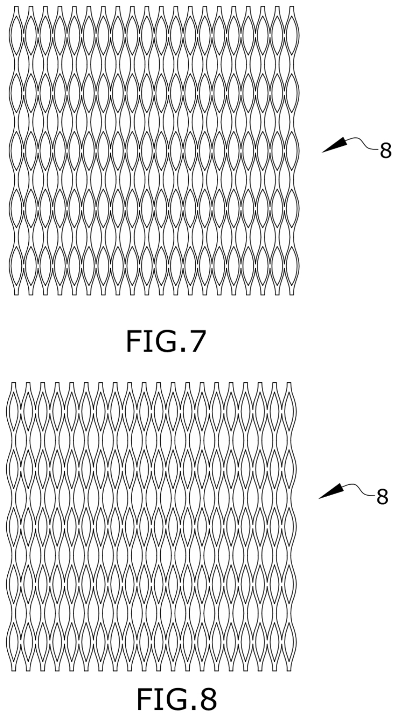

As a result of joining the unit cells (3) at their intermediate points (7) with other adjacent unit cells, and at their upper (5) and lower (6) attachment points with unit cells arranged in rows above and below, an array (8) is formed, wherein the array (8) shown in figure 7 has less stiffness than the array shown in figure 8 as it is formed by unit cells with the arms (3.3) and (3.4) with less thickness.

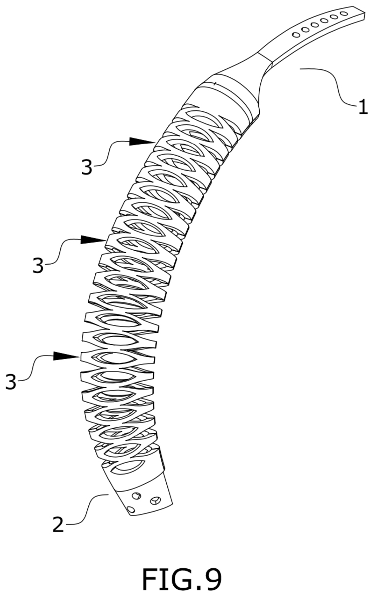

Figure 9 shows a rib prosthesis (3) in which the cells closer to the front end (1) are thicker than the cells closer to the rear end (2), so that the elasticity is progressive, generating greater local stiffness near the front end (1).

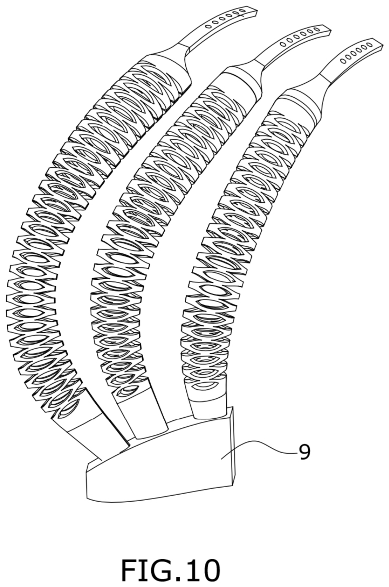

Although the embodiment shown in the figures explains what the prosthesis of a single rib would be like, the same inventive concept could also be used for more than one rib or even for the reconstruction of a part of the rib cage, as shown in figure 10, wherein several rib prostheses are joined at one end to a sternal prosthesis element (9) forming part of the rib cage, since what is really inventive is the fact of having controlled flexibility, thanks to the constructive characteristics of the array and of the unit cells (3).

Having sufficiently described the nature of the present invention as well as the manner of putting it into practice, it should be noted that, within its essential nature, it may be put into practice in other embodiments that differ in detail from the embodiment indicated by way of example, and to which the protection claimed will also apply, provided that it does not alter, change or modify its fundamental principle.

## Claims

1. A rib prosthesis with controlled elasticity, **characterised in that** it comprises an front end (1) and a rear end (2) arranged opposite each other and, between them, an intermediate elastic structure capable of absorbing pressure in all directions and consisting of an array (8) of unit cells (3) with an inner cavity (4), the unit cells being joined to the adjacent cells by means of intermediate attachment points (7), by means of upper attachment points (5) and other lower attachment points (6).

2. The rib prosthesis with controlled elasticity according to claim 1, **characterised in that** each of the unit cells (3), seen from a flat configuration, is formed by a first arm (3.3) and a second arm (3.4) opposite each other in such a way that a central space (3.5) is defined, the upper ends of the first arm (3.3) and of the second arm (3.4) being joined to form an upper joint (3.1), while the lower ends of the first arm (3.3) and of the second arm (3.4) are joined to form a lower joint (3.2).

3. The rib prosthesis with controlled elasticity according to claim 2, **characterised in that** depending on the thickness of the first arm (3.3) and of the second arm (3.4) of the unit cells (3), as well as the internal angle (3.6) defined at the ends of the central spaces (3.5), different elasticity is achieved.

4. A thoracic prosthesis according to any of the preceding claims, **characterised in that** it uses one or more rib prostheses joined at one or both ends to a common fixing element (9).
